# EUROPEAN PATENT SPECIFICATION

(11) **EP 3 308 713 B1**
(45) Date of publication and mention of the grant of the patent: **06.05.2020**
(21) Application number: 15894868.7
(22) Date of filing: 12.06.2015
(51) Int. Cl.: A61B 8/08, A61B 5/20, A61B 5/107, A61B 5/00

(54) **URINE AMOUNT ESTIMATION DEVICE AND URINE AMOUNT ESTIMATION METHOD**
VORRICHTUNG ZUR KALKULATION EINER URINMENGE UND VERFAHREN ZUR KALKULATION EINER URINMENGE
DISPOSITIF D'ESTIMATION DE QUANTITÉ D'URINE ET MÉTHODE D'ESTIMATION DE QUANTITÉ D'URINE

(43) Date of publication of application: 18.04.2018
(73) Proprietor: Triple W Japan K.K., Tokyo 1500031 (JP)
(72) Inventor: NAKANISHI, Atsushi, Tokyo 1500031 (JP); MASAMORI, Ryosuke, Tokyo 1500031 (JP)
(74) Representative: advotec.
(86) International application number: PCT/JP2015/002966
(87) International publication number: WO 2016/199182

(56) References cited:
- EP-A1- 1 731 101
- EP-A2- 0 271 214
- CN-A- 102 178 550
- JP-A- H07 171 149
- JP-A- 2007 075 383
- JP-A- 2014 023 813
- JP-B2- H0 444 547
- US-A1- 2007 123 778
- US-A1- 2011 004 123
- US-B1- 6 213 949

## Description

### TECHNICAL FIELD

The art disclosed herein relates to a urine amount estimation device and urine amount estimation method.

### BACKGROUND ART

Disclosed at Patent Reference No. 1 is art for estimating the amount of urine in the bladder based on the distance between the anterior wall and the posterior wall of the bladder.

### PRIOR ART REFERENCES

### PATENT REFERENCES

PATENT REFERENCE NO. 1: Japanese Patent No. 4677615

In US 2011/004123 A1 a system and method are provided for monitoring bladder distention. Independently controllable acoustic transmitters are coupled to the surface of a user's abdomen region in a spaced-apart fashion. Each transmitter can introduce a broadband acoustic signal into a portion of the abdomen region. Acoustic receivers are also coupled to the surface of the abdomen region to detect acoustic reflections generated when the acoustic signals are introduced into the abdomen region. A controller is positioned on the user and is correlated to an orientation of the user. The controller stores a profile indicative of anatomical features of the user. The controller initiates periodic activations of selected ones of the transmitters based upon the profile and the orientation of the user. The controller uses a weighted combination of all of the acoustic reflections to determine bladder distention of the user. The weighted combination is defined by at least the profile and orientation of the user.

### SUMMARY OF INVENTION

### PROBLEM TO BE SOLVED BY INVENTION

However, there are situations in which the anterior wall and the posterior wall of the bladder cannot be detected properly, in which case accurate estimation of the amount of urine in the bladder will not be possible. In other words, the art of Patent Reference No. 1 requires that both the anterior wall and the posterior wall of the bladder be detected with high precision.

The art disclosed herein was conceived in light of such points, it being an object thereof to facilitate estimation of the amount of urine in the bladder.

### MEANS FOR SOLVING PROBLEM

A urine amount estimation device disclosed herein comprises a sensor that transmits an ultrasonic wave into the body and that detects a reflected wave produced by said ultrasonic wave; and an estimating unit that estimates an amount of urine in a bladder based on whether a reflected wave from the bladder which is detected by the sensor is present and/or based on a magnitude of the reflected wave from the bladder which is detected by the sensor.

Preferably, a urine amount estimation device comprises a sensor that transmits an ultrasonic wave into the body and that detects a reflected wave produced by said ultrasonic wave; and an estimating unit that estimates an amount of urine in a bladder based on whether a reflected wave from a small intestine which is detected by the sensor is present and/or based on a magnitude of the reflected wave from the small intestine which is detected by the sensor.

Preferably, a urine amount estimation method comprises an operation in which an ultrasonic wave is transmitted into an interior of a body; an operation in which a wave from the interior of the body produced by reflection of said ultrasonic wave is detected; and an operation in which an amount of urine in a bladder is estimated based on whether a detected reflected wave from the bladder is present and/or based on a magnitude of the detected reflected wave from the bladder.

A urine amount estimation method disclosed herein comprises an operation in which an ultrasonic wave is transmitted into an interior of a body; an operation in which a wave from the interior of the body produced by reflection of said ultrasonic wave is detected; and an operation in which an amount of urine in a bladder is estimated based on whether a detected reflected wave from a small intestine is present and/or based on a magnitude of the detected reflected wave from the small intestine.

### BENEFIT OF INVENTION

The urine amount estimation device makes it possible to facilitate estimation of the amount of urine.

The urine amount estimation method makes it possible to facilitate estimation of the amount of urine.

### BRIEF DESCRIPTION OF DRAWINGS

[FIG. 1] FIG. 1 is a block diagram of a urine amount estimation device.
[FIG. 2] FIG. 2 is a diagram illustrating how a urine amount estimation device is used.
[FIG. 3] FIG. 3 is a perspective view of urine amount estimation device 100.
[FIG. 4] FIG. 4 is a flowchart of determination of when it is time to urinate.
[FIG. 5] FIG. 5 is a schematic sectional view centered on the lower abdomen of a human body at a time when there is a small amount of urine.
[FIG. 6] FIG. 6 is one example of a received signal at a time when there is a small amount of urine.
[FIG. 7] FIG. 7 is a schematic sectional view centered on the lower abdomen of a human body at a time when there is a moderate amount of urine.
[FIG. 8] FIG. 8 is one example of a received signal at a time when there is a moderate amount of urine.
[FIG. 9] FIG. 9 is a schematic sectional view centered on the lower abdomen of a human body at a time when there is a large amount of urine.
[FIG. 10] FIG. 10 is one example of a received signal at a time when there is a large amount of urine.
[FIG. 11] FIG. 11 is a flowchart of determination of when it is time to urinate in accordance with a variation.
[FIG. 12] FIG. 12 is one example of a received signal that has been subjected to [FIG. 13] FIG. 13 is one example of a received signal that has been subjected to signal processing similar to that at FIG. 12, but at FIG. 13 the location at which ultrasonic sensor 1 is worn is higher than is the case at FIG. 12.

### EMBODIMENTS FOR CARRYING OUT INVENTION

Below, illustrative examples in the form of embodiments are described in detail with reference to the drawings.

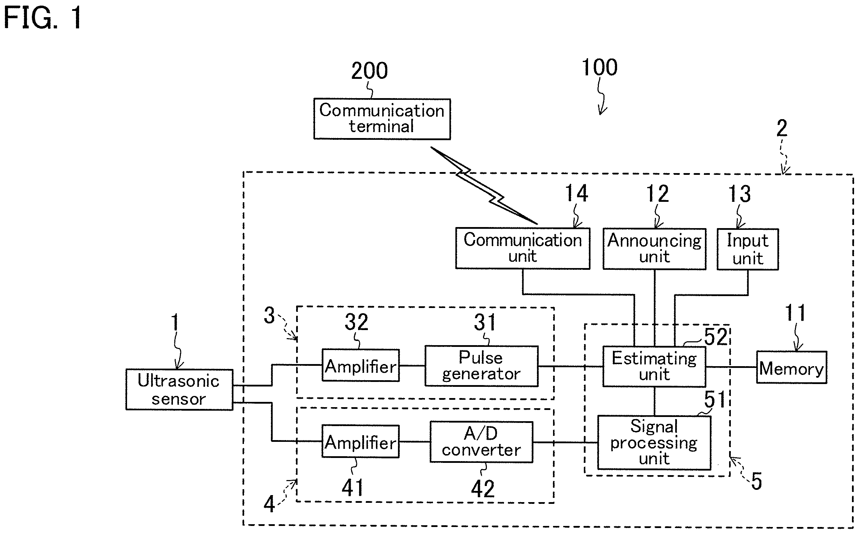

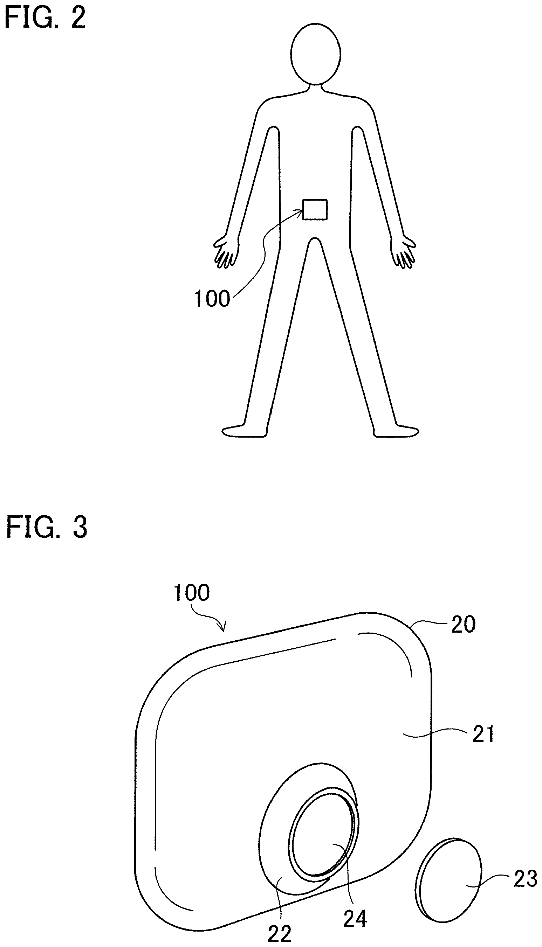

FIG. 1 shows a block diagram of urine amount estimation device 100. FIG. 2 shows a drawing for explaining how urine amount estimation device 100 is used. FIG. 3 shows a perspective view of urine amount estimation device 100.

Urine amount estimation device 100 is for estimating the amount of urine that has collected in the bladder of a subject. For example, the subject might be an elderly person, a physically disabled person or other such person requiring nursing care, a person not requiring nursing care but requiring time before the person is ready to use the toilet due to a physical handicap, or the like. Note, however, that subjects are not limited to the foregoing. Urine amount estimation device 100 is provided with ultrasonic sensor 1 and with device body 2 which controls ultrasonic sensor 1. As shown in FIG. 2, urine amount estimation device 100 is worn by the subject. At least ultrasonic sensor 1 is arranged at a location which is over the skin of the abdomen of the subject and which corresponds to the bladder (e.g., the lower abdomen). Ultrasonic sensor 1 and device body 2 are housed within case 20, being constituted in integral fashion.

As shown in FIG. 3, case 20 is formed in planar and more or less rectangular fashion. That surface (hereinafter "contacting surface") 21 of case 20 which comes in contact with the abdomen of the subject is provided with protrusion 22, ultrasonic sensor 1 being incorporated within protrusion 22. At contacting surface 21, protrusion 22 is arranged at location which is in the approximate center in the width direction and which is comparatively low in the vertical direction (below the center in the vertical direction). Provided at the tip of protrusion 22 is gel pad 23 for improving penetration of ultrasonic waves with respect to the abdomen. More specifically, concavity 24 is formed at the tip of protrusion 22, concavity 24 being provided with gel pad 23. Protrusion 22 contacts the abdomen of the subject by way of gel pad 23 which intervenes therebetween.

By wrapping a belt about the abdomen in such fashion as to pass over urine amount estimation device 100, urine amount estimation device 100 is worn by the subject in such a manner as to cause contacting surface 21 to oppose the abdomen of the subject and cause protrusion 22 to come in contact with the skin. Alternatively, by causing adhesive tape that passes over urine amount estimation device 100 to adhere to the abdomen, urine amount estimation device 100 is worn by the subject in such a manner as to cause contacting surface 21 to oppose the abdomen of the subject and cause protrusion 22 to come in contact with the skin.

Ultrasonic sensor 1 transmits and receives ultrasonic waves. More specifically, ultrasonic sensor 1 has transducer(s) constituted from piezoelectric element(s). Whereas ultrasonic sensor 1 generates an ultrasonic wave upon oscillating in response to a drive voltage, it also generates an electrical signal in response to an oscillation produced upon receiving an ultrasonic wave. Ultrasonic sensor 1 is an example of a sensor.

As shown in FIG. 1; device body 2 has transmitter 3 which outputs drive voltage to ultrasonic sensor 1; receiver 4 which receives electrical signals from ultrasonic sensor 1; controller 5 which carries out overall control of urine amount estimation device 100 and estimates the amount of urine; memory 11; announcing unit 12 for announcing various types of information to the exterior; input unit 13 for carrying out input of the fact that urination has actually occurred; and communication unit 14 for carrying out communication with the exterior.

Transmitter 3 supplies drive voltage to ultrasonic sensor 1. Transmitter 3 has pulse generator 31 and amplifier 32. Pulse generator 31 generates a pulsed signal of prescribed pulsewidth and voltage. Pulse generator 31 may be constituted such that pulsewidth and/or frequency are variable. Amplifier 32 amplifies the pulsed signal from pulse generator 31 and outputs this to ultrasonic sensor 1 in the form of a drive voltage.

Receiver 4 receives an electrical signal from ultrasonic sensor 1. Receiver 4 has amplifier 41 and A/D converter 42. Amplifier 41 amplifies the received signal which is sent thereto from ultrasonic sensor 1, and outputs this to A/D converter 42. A/D converter 42 carries out A/D conversion of the received signal which is sent thereto from amplifier 41, and outputs this to controller 5.

Announcing unit 12 might, for example, be a vibrator. As a result of vibration of the vibrator, various types of information (e.g., the fact that it is time to urinate) are announced to the subject.

Input unit 13 is an actuatable unit for allowing a user to cause various types of input (e.g., input of the fact that urination has actually occurred) to be made to urine amount estimation device 100. For example, input unit 13 might be a pushbutton.

Communication unit 14 carries out communication with an external communication terminal. External communication terminal(s) with which communication is to be carried out can be registered with urine amount estimation device 100, information related to said communication terminal(s) being stored at memory 11. That is, a user might register communication terminal 200 with urine amount estimation device 100 in advance. By so doing, this will make it possible for communication to take place between urine amount estimation device 100 and communication terminal 200. For example, a communication terminal 200 in the possession of a nursing caregiver might be registered with urine amount estimation device 100. Furthermore, where the subject is able to use the toilet unassisted, a communication terminal 200 belonging to the subject might be registered therewith. This may be such that only one communication terminal 200 is registrable therewith or this may be such that multiple communication terminals 200 (e.g., a communication terminal belonging to the nursing caregiver and a communication terminal belonging to the person requiring nursing care) are registrable therewith.

Controller 5 has one or more processors. Controller 5 controls transmitter 3 to output a drive voltage to ultrasonic sensor 1, and also estimates urine amount based on the received signal which is sent thereto from receiver 4. Controller 5 has signal processing unit 51 which performs signal processing on the received signal that is sent thereto from receiver 4, and estimating unit 52 which controls transmitter 3 and also estimates urine amount.

Signal processing unit 51 performs averaging and/or other such signal processing on the received signal which is input thereto from receiver 4, and outputs the processed signal to estimating unit 52.

Estimating unit 52 outputs a pulsed signal generation instruction to pulse generator 31 of transmitter 3, and estimates urine amount based on the received signal which is input thereto from signal processing unit 51. Note that control of transmitter 3 may be carried out by a component other than estimating unit 52.

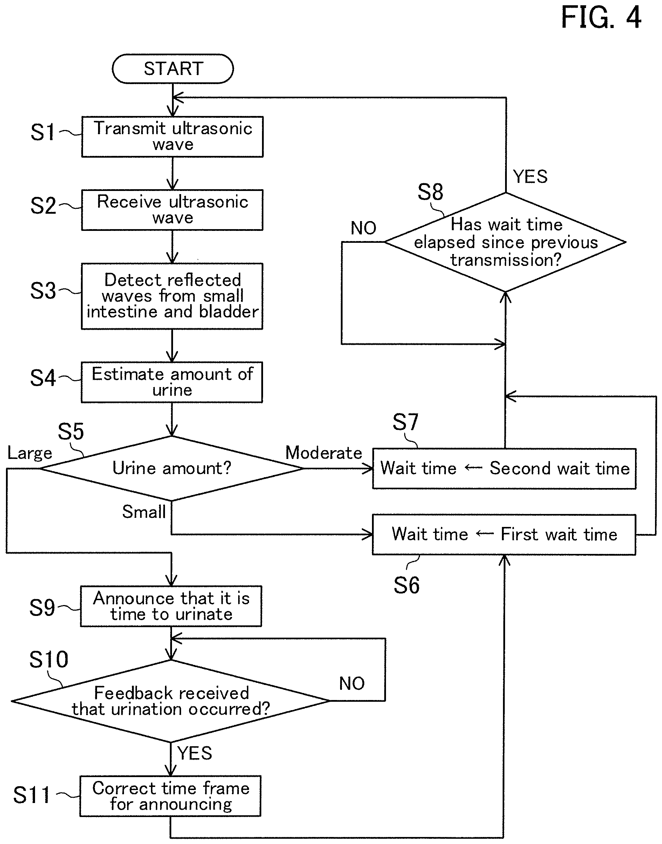

Processing at estimating unit 52 is described below using the flowchart shown in FIG. 4.

First, estimating unit 52 outputs an ultrasonic wave transmit instruction (step S1). More specifically, estimating unit 52 outputs a pulsed signal generation instruction to transmitter 3. This generation instruction serves as trigger for processing to estimate urine amount. Transmitter 3 supplies a drive voltage based on the generation instruction to ultrasonic sensor 1. Ultrasonic sensor 1 transmits an ultrasonic wave based on the drive voltage. Estimating unit 52 outputs a generation instruction with prescribed periodicity.

Next, when ultrasonic sensor 1 receives an ultrasonic wave (i.e., the received signal is input to signal processing unit 51) (step S2), estimating unit 52 detects a reflected wave from the bladder and a reflected wave from the small intestine based on said received signal (step S3). In addition, estimating unit 52 estimates the amount of urine in the bladder based on the reflected wave from the bladder and the reflected wave from the small intestine (step S4).

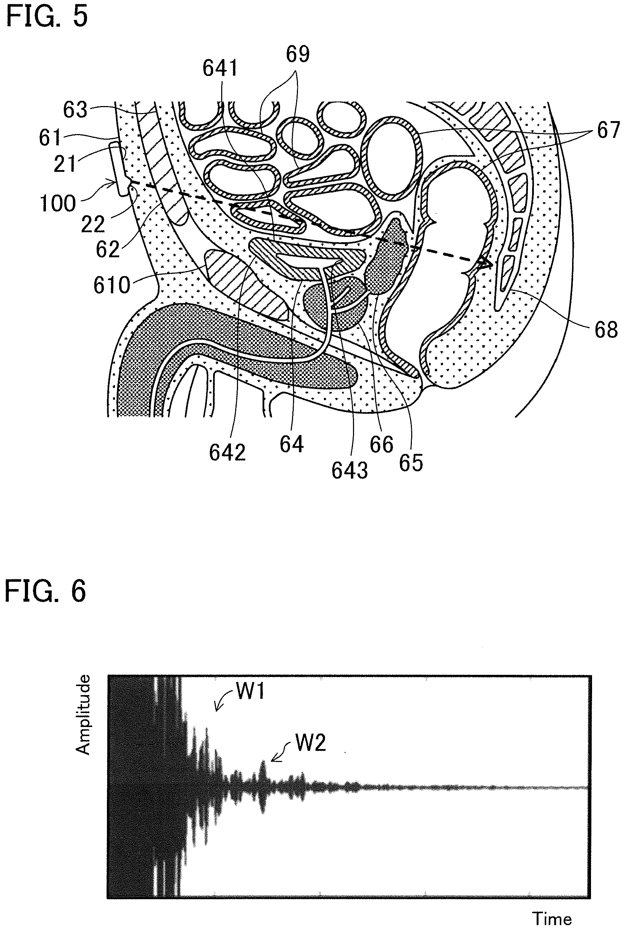

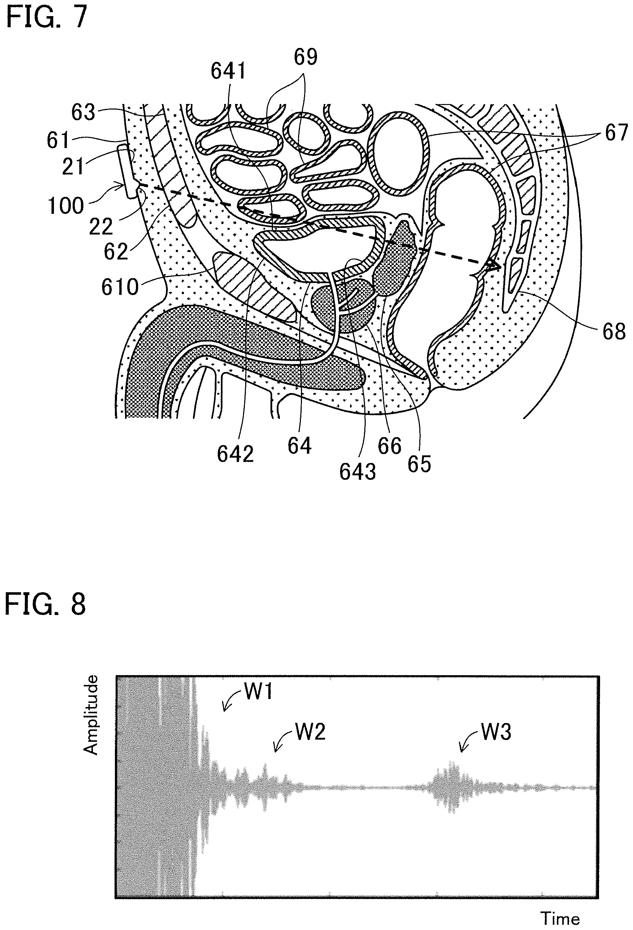

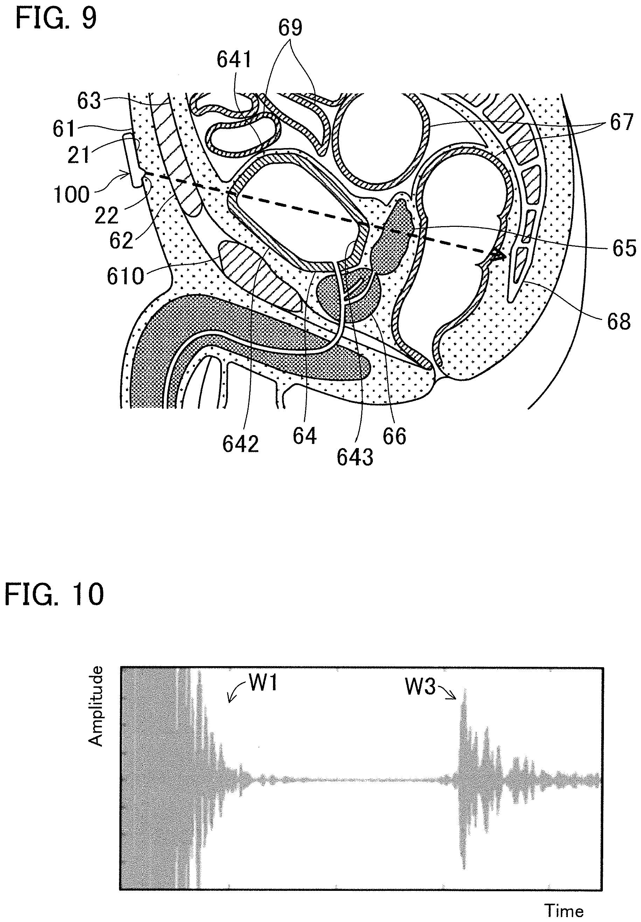

Here, estimation of the amount of urine in the bladder based on the reflected wave from the bladder and the reflected wave from the small intestine is described with reference to FIGS. 5 through 10. FIG. 5 shows a schematic sectional view of the lower abdomen of a human body at a time when there is a small amount of urine. FIG. 6 shows an example of a waveform of a received signal at a time when there is a small amount of urine, the situation shown corresponding to that shown in FIG. 5. FIG. 7 shows a schematic sectional view of the lower abdomen of a human body at a time when there is a moderate amount of urine. FIG. 8 shows an example of a waveform of a received signal at a time when there is a moderate amount of urine, the situation shown corresponding to that shown in FIG. 7. FIG. 9 shows a schematic sectional view of the lower abdomen of a human body at a time when there is a large amount of urine. FIG. 10 shows an example of a waveform of a received signal at a time when there is a large amount of urine, the situation shown corresponding to that shown in FIG. 9. The received signals shown at FIGS. 6, 8, and 10 undergo signal processing at signal processing unit 51.

As shown in FIG. 5, arranged in order in a line from the front of the abdomen to the back are subcutaneous fat 61, muscle 62, fat 63, bladder 64, seminal vesicle 65 or prostate gland 66 (in the case of a male) or vagina (in the case of a female), rectum 67, spinal column (sacrum) 68, and so forth. Disposed above the bladder 64 is the small intestine 69, and disposed diagonally downward from the front of the bladder 64 is the pubic bone 610. As is clear from FIGS. 5, 7, and 9, increase in the amount of urine causes the bladder 64 to expand upward. At such time, the bladder 64 pushes upward on the small intestine 69.

As is clear from FIG. 6, when there is a small amount of urine in the bladder, reflected wave W1 and reflected wave W2 are observed. Noise is observed immediately following transmission of the ultrasonic wave. Reflected wave W1, which is observed immediately following the noise, is the wave reflected from the abdominal subcutaneous fat 61, muscle 62, fat 63, and so forth.

Reflected wave W2 is the wave reflected from the small intestine 69. Because peristalsis is constantly taking place at the small intestine 69, reflected wave W2 does not appear as a distinct peak but as a noise-like signal. As shown in FIG. 5, when there is a small amount of urine in the bladder 64, the bladder 64 shrinks to a small size, and small intestine 69 which is disposed above the bladder 64 moves downward to occupy a comparatively low position in the lower abdomen. The small intestine 69 will at such a time come to be disposed right along the propagation path (broken line at FIGS. 5, 7, and 9) of the ultrasonic wave that is emitted from urine amount estimation device 100 which is worn around the lower abdomen. When there is a small amount of urine in the bladder, reflected wave W2 from the small intestine 69 is therefore observed.

Note that because gas is mixed with the contents at the interior of the small intestine 69, the ultrasonic wave is attenuated by the small intestine 69 and tends not to reach deep into the body. For this reason, at the received signal in FIG. 6, a conspicuous reflected wave is not observed after reflected wave W2.

As shown in FIG. 7, as the amount of urine in the bladder 64 increases, the bladder 64 expands upward, and the small intestine 69 which is disposed along the propagation path of the ultrasonic wave gradually moves upward. When there is a moderate amount of urine in the bladder 64, the small intestine 69 and the bladder 64 come to be disposed along the propagation path of the ultrasonic wave.

For this reason, as shown in FIG. 8, when there is a moderate amount of urine, in addition to reflected wave W1 and reflected wave W2, reflected wave W3 is observed after reflected wave W2. Reflected wave W3 is the wave reflected from the posterior wall 643 of the bladder 64. More particularly, reduction of the portion of the small intestine 69 that lies along the propagation path causes the ultrasonic wave to reach deeper into the body, as a result of which reflected wave W3 from the posterior wall 643 of the bladder 64 comes to be observed. Furthermore, because of the reduction in the portion of the small intestine 69 that lies along the propagation path, the amplitude of reflected wave W2 from the small intestine 69 is smaller than when there was a small amount of urine (FIG. 6).

Moreover, as shown in FIG. 9, increase in the amount of urine in the bladder 64 causes the bladder 64 to expand upward further, and the portion of the small intestine 69 which is disposed along the propagation path of the ultrasonic wave becomes almost completely nonexistent. This causes the ultrasonic wave to reach deeper into the body.

For this reason, as shown in FIG. 10, when there is a large amount of urine, reflected wave W2 from the small intestine 69 is almost completely unobserved. Furthermore, amplitude of reflected wave W3 from the posterior wall 643 of the bladder 64 becomes large.

It is thus possible to estimate the amount of urine based on at least one of: whether reflected wave W2 from the small intestine 69 is present; the magnitude of reflected wave W2 from the small intestine 69; whether reflected wave W3 from the posterior wall 643 of the bladder 64 is present; and the magnitude of reflected wave W3 from the posterior wall 643 of the bladder 64.

In accordance with one example, estimating unit 52 might estimate amount of urine based on whether reflected wave W2 from the small intestine 69 is present and based on whether reflected wave W3 from the posterior wall 643 of the bladder 64 is present. Because the receive time bands during which reflected wave W2 and reflected wave W3 can be expected to arrive following reflection are more or less known, estimating unit 52 determines whether the reflected waves are observed during the respective receive time bands for reflected wave W2 and reflected wave W3. Based on the combination of whether reflected wave W2 is present and whether reflected wave W3 is present, estimating unit 52 estimates amount of urine in three steps as being "small," "moderate," or "large." More specifically, when reflected wave W2 is observed but reflected wave W3 is not observed, estimating unit 52 estimates that amount of urine is "small." When reflected wave W2 and reflected wave W3 are observed, estimating unit 52 estimates that amount of urine is "moderate." When reflected wave W2 is not observed but reflected wave W3 is observed, estimating unit 52 estimates that amount of urine is "large." The state in which the amount of urine is "small" corresponds to a first state, the state in which the amount of urine is "moderate" corresponds to a second state, and the state in which the amount of urine is "large" corresponds to a third state. Note that whether reflected wave W2 is present may be determined not based on whether the amplitude of reflected wave W2 is exactly zero, it being possible to determine that reflected wave W2 is present when the amplitude of reflected wave W2 is greater than or equal to a prescribed threshold value, and to determine that reflected wave W2 is absent when the amplitude of reflected wave W2 is less than the prescribed threshold value. And the same thing is true for reflected wave W3.

Next, estimating unit 52 determines when it is or will be time to urinate based on the estimated amount of urine (step S5). More specifically, when the amount of urine is "small," estimating unit 52 determines that it is not yet time to urinate, and also causes a prescribed first wait time to be set as the wait time (step S6). In addition, estimating unit 52 determines whether the first wait time has elapsed since the previous ultrasonic wave transmission (step S8). If the first wait time has elapsed, processing returns to step S1, where estimating unit 52 carries out transmission of an ultrasonic wave. On the other hand, if the first wait time has not elapsed, estimating unit 52 waits for the first wait time to elapse, processing returning to step S1 after the first wait time has elapsed. In this way, when the amount of urine is "small," estimating unit 52 carries out transmission of ultrasonic waves at intervals equal to the first wait time, and periodically carries out determination of whether it is time to urinate. In other words, the first wait time corresponds to the period at which estimation of the amount of urine is carried out in repeated fashion when the amount of urine is "small."

When the amount of urine is "moderate," estimating unit 52 determines that it is not yet time to urinate, and also causes a second wait time, which is shorter than the first wait time, to be set as the wait time (step S7). In addition, estimating unit 52 determines whether the second wait time has elapsed since the previous ultrasonic wave transmission (step S8). If the second wait time has elapsed, processing returns to step S1, where estimating unit 52 carries out transmission of an ultrasonic wave. On the other hand, if the second wait time has not elapsed, estimating unit 52 waits for the second wait time to elapse, processing returning to step S1 after the second wait time has elapsed. In this way, when the amount of urine is "moderate," estimating unit 52 carries out transmission of ultrasonic waves at intervals equal to the second wait time, and periodically carries out determination of whether it is time to urinate. In other words, the second wait time corresponds to the period at which estimation of the amount of urine is carried out in repeated fashion when the amount of urine is "moderate."

When the amount of urine is "large," estimating unit 52 determines that it is time to urinate, and announces that it is time to urinate (step S9). More specifically, estimating unit 52 causes operation of announcing unit 12, and also announces, to registered communication terminal(s) 200 by way of communication unit 14, that it is time to urinate. As a result, because the subject is given advance notice of the approaching need to urinate, this permits the subject to make preparations to use the toilet.

Furthermore, as a result of downloading of a special-purpose software application for urine amount estimation device 100, it is possible for communication terminal 200 to communicate with urine amount estimation device 100 and to actuate urine amount estimation device 100. Upon receipt of a signal from urine amount estimation device 100 indicating that it is time to urinate, communication terminal 200 causes a message indicating that it is time to urinate to be shown to the user at a display and/or otherwise announces to the owner of communication terminal 200 that it is time to urinate. As a result, the owner of communication terminal 200 is informed that it is time to urinate, making it possible for prompting to occur with respect to inducement of the subject to use the toilet.

Thus, when the amount of urine is small, estimating unit 52 carries out estimation of the amount of urine in repeated fashion at intervals equal to the first wait time. Upon increase in the amount of urine, when the amount of urine becomes moderate, estimating unit 52 carries out estimation of the amount of urine in repeated fashion at intervals equal to the second wait time, which is shorter than the first wait time. In other words, the period at which estimating unit 52 carries out estimation of the amount of urine in repeated fashion is shorter. Upon further increase in the amount of urine, when the amount of urine becomes large, estimating unit 52 determines that it is time to urinate, and announces to the subject that it is time to urinate. By increasing the frequency with which estimation of the amount of urine is carried out in accompaniment to increase in the amount of urine, it is possible to improve the precision with which determination that it is time to urinate is carried out.

Furthermore, each time that determination of whether it is time to urinate (i.e., the series of processing steps from the pulsed signal generation instruction to the determination) is periodically executed, estimating unit 52 stores the amount of urine at memory 11. In other words, the change in the amount of urine as a function of time is stored in memory 11.

Then, after having announced that it is time to urinate, estimating unit 52 waits for report of the fact that urination has actually occurred (step S10). Following completion of actual urination, actuation of input unit 13 might be performed by the subject or a nursing caregiver or other third party. Moreover, the fact that urination has actually occurred may also be input to urine amount estimation device 100 from communication terminal 200. When actual urination has been completed, the owner of communication terminal 200 would actuate communication terminal 200 and transmit to urine amount estimation device 100 the fact that urination has actually occurred.

Upon receiving input of report of the fact that urination has actually occurred from communication terminal 200 or a signal from input unit 13, estimating unit 52 determines that urination has actually occurred and carries out correction of the time frame for announcing when it is time to urinate (step S11). For example, estimating unit 52 might determine whether the time frame for announcing is appropriate based on the time from when it announced that it was time to urinate until when it received input of the fact that urination had actually occurred. If the difference between the time from announcing that it is time to urinate until input of the fact that urination has actually occurred and the assumed time allowed for urination to occur following announcement of the fact that it is time to urinate is within a prescribed range, estimating unit 52 might cause the time frame for announcing to be left unchanged. This is because, in such a situation, it would be the case that estimating unit 52 is employing an appropriate time frame for announcing when it is time to urinate. On the other hand, if the difference between the time from announcing that it is time to urinate until input of the fact that urination has actually occurred is greater than the assumed time allowed for urination to occur by more than the prescribed range, estimating unit 52 might cause the time frame for announcing to be modified so that announcement occurs at a later point in time. More specifically, following determination that the amount of urine is "large," estimating unit 52 waits a short time, thereafter announcing that it is time to urinate. The time frame for announcing might be adjusted as a result of adjustment of this wait time. Or if the difference between the time from announcing that it is time to urinate until input of the fact that urination has actually occurred is less than the assumed time allowed for urination to occur by more than the prescribed range, estimating unit 52 might cause the time frame for announcing to be modified so that announcement occurs at an earlier point in time. More specifically, to cause estimating unit 52 to determine that the amount of urine is "large" at an earlier point in time, the threshold value for determining whether reflected wave W2 is present might be increased. In other words, this will cause the determination that reflected wave W2 is not present to be made at an earlier point in time, which will cause the time frame for announcing to be modified so that announcement occurs at an earlier point in time.

Estimating unit 52 causes the threshold value and wait time as corrected at step S11 to overwrite those at memory 11. The storage capacity of the bladder varies from person to person. At a time when urination has actually occurred, receipt of feedback and correction of the time frame for announcing makes it possible to announce when it is time to urinate at an appropriate time frame in correspondence to the subject.

Following completion by estimating unit 52 of correction of the threshold value for determination, processing proceeds to step S6. In other words, after the time to urinate has passed, estimating unit 52 again causes the wait time to be set to the first wait time which corresponds to the situation that exists when the amount of urine is "small," carries out transmission of ultrasonic waves at intervals equal to the first wait time, and periodically carries out determination of whether it is time to urinate.

As described above, urine amount estimation device 100 is provided with ultrasonic sensor 1 which transmits ultrasonic waves into the body and which detects waves produced by reflection of said ultrasonic waves; and estimating unit 52 which estimates the amount of urine in the bladder based on reflected wave W3 from the bladder as detected by ultrasonic sensor 1. Stating this another way, a urine amount estimation method employed by urine amount estimation device 100 comprises an operation in which an ultrasonic wave is transmitted into the interior of the body; an operation in which a wave from the interior of the body produced by reflection of said ultrasonic wave is detected; and an operation in which an amount of urine in the bladder is estimated based on the detected reflected wave from the bladder.

Because the shape and/or size of the bladder varies in correspondence to the amount of urine in the bladder, reflected wave W3 from the bladder also varies in correspondence to the amount of urine in the bladder. Estimating unit 52 therefore estimates the amount of urine in the bladder based on reflected wave W3 from the bladder. This makes it possible for estimating unit 52 to estimate the amount of urine in the bladder in noninvasive fashion.

More specifically, estimating unit 52 is such that whereas it is estimated that the amount of urine in the bladder is small when reflected wave W3 from the bladder is not detected by ultrasonic sensor 1, it is estimated that the amount of urine in the bladder is large when reflected wave W3 from the bladder is detected by ultrasonic sensor 1.

The greater the amount of urine therein the larger the bladder will be. In other words, when the amount of urine is small, because the bladder is small, it is sometimes the case that reflected wave W3 from the bladder does not return to the location from which the ultrasonic wave was transmitted into the body. On the other hand, after the amount of urine has become large, because the bladder is large, there is a greater tendency for reflected wave W3 from the bladder to return to the location from which the ultrasonic wave was transmitted into the body. For this reason, estimating unit 52 is such that whereas it is estimated that the amount of urine in the bladder is small when reflected wave W3 from the bladder is not detected, it is estimated that the amount of urine in the bladder is large when reflected wave W3 from the bladder is detected. In this way, estimating unit 52 estimates amount of urine based on whether reflected wave W3 from the bladder is present.

Furthermore, urine amount estimation device 100 is provided with ultrasonic sensor 1 which transmits ultrasonic waves into the body and which detects waves produced by reflection of said ultrasonic waves; and estimating unit 52 which estimates the amount of urine in the bladder based on reflected wave W2 from the small intestine as detected by ultrasonic sensor 1. Stating this another way, a urine amount estimation method employed by urine amount estimation device 100 comprises an operation in which an ultrasonic wave is transmitted into the interior of the body; an operation in which a wave from the interior of the body produced by reflection of said ultrasonic wave is detected; and an operation in which an amount of urine in the bladder is estimated based on the detected reflected wave from the small intestine.

As has been described above, the shape and/or size of the bladder varies in correspondence to the amount of urine in the bladder. Because the small intestine is disposed above the bladder, the location of the small intestine also varies in correspondence to the amount of urine in the bladder. In other words, reflected wave W2 from the small intestine also varies in correspondence to the amount of urine in the bladder. Estimating unit 52 therefore estimates the amount of urine in the bladder based on reflected wave W2 from the small intestine 69. This makes it possible for estimating unit 52 to estimate the amount of urine in the bladder in noninvasive fashion.

More specifically, estimating unit 52 is such that whereas it is estimated that the amount of urine in the bladder is small when reflected wave W2 from the small intestine is detected by ultrasonic sensor 1, it is estimated that the amount of urine in the bladder is large when reflected wave W2 from the small intestine is not detected by ultrasonic sensor 1.

The greater the amount of urine therein the more that the bladder will expand upward. In other words, when the amount of urine is small, the bladder shrinks to a small size and is disposed at a location toward the bottom of the lower abdomen. For this reason, in correspondence to the bladder, the small intestine is disposed at a comparatively low position in the lower abdomen. In contrast, when the amount of urine is large, the bladder expands upward, and the small intestine also moves upward from the lower abdomen to occupy a higher position. For this reason, in a situation in which ultrasonic sensor 1 is worn on the lower abdomen, there is a tendency for reflected wave W2 from the small intestine to return thereto when the amount of urine is small, and there is a tendency for reflected wave W2 from the small intestine not to return thereto when the amount of urine is large. For this reason, estimating unit 52 is such that whereas it is estimated that the amount of urine in the bladder is small when reflected wave W2 from the small intestine is detected, it is estimated that the amount of urine in the bladder is large when reflected wave W2 from the small intestine is not detected. In this way, estimating unit 52 estimates amount of urine based on whether reflected wave W2 from the small intestine is present.

Moreover, estimating unit 52 estimates amount of urine based on both reflected wave W3 from the bladder as detected by ultrasonic sensor 1 and reflected wave W2 from the small intestine as detected by ultrasonic sensor 1.

In other words, as has been described above, reflected wave W3 from the bladder and reflected wave W2 from the small intestine respectively vary in correspondence to the amount of urine in the bladder. Furthermore, reflected wave W3 from the bladder and reflected wave W2 from the small intestine respectively display different behavior as a function of the amount of urine in the bladder. Estimating unit 52 therefore estimates the amount of urine in the bladder in such fashion that both reflected wave W3 from the bladder and reflected wave W2 from the small intestine are taken into consideration. This makes it possible for estimating unit 52 to more precisely estimate the amount of urine in the bladder.

More specifically, estimating unit 52 estimates that a first state, for which the amount of urine in the bladder is small, exists when reflected wave W3 from the bladder is not detected by ultrasonic sensor 1 but reflected wave W2 from the small intestine is detected by ultrasonic sensor 1; estimates that a second state, for which the amount of urine in the bladder is greater than the first state, exists when reflected wave W3 from the bladder is detected by ultrasonic sensor 1 and reflected wave W2 from the small intestine is detected by ultrasonic sensor 1; and estimates that a third state, for which the amount of urine in the bladder is greater than the second state, exists when reflected wave W3 from the bladder is detected by ultrasonic sensor 1 but reflected wave W2 from the small intestine is not detected by ultrasonic sensor 1.

This constitution makes it possible for the amount of urine in the bladder to be estimated through combination of reflected wave W3 from the bladder and reflected wave W2 from the small intestine. For this reason, this makes it possible to estimate the amount of urine in the bladder more precisely than is the case either when the amount of urine in the bladder is estimated with only reflected wave W3 from the bladder or when the amount of urine in the bladder is estimated with only reflected wave W2 from the small intestine.

Estimating unit 52 causes the frequency with which reflected waves are detected by ultrasonic sensor 1 to be greater when the estimated amount of urine is large than when the estimated amount of urine is small.

When the amount of urine is small, because the urgency to detect when it is time to urinate is low, estimating unit 52 causes detection frequency to be reduced. This will make it possible to suppress electrical power consumption. On the other hand, when the amount of urine has increased, because the urgency to detect when it is time to urinate will also have increased, estimating unit 52 causes detection frequency to be increased. This will make it possible to always be able to detect when it is time to urinate.

More specifically, estimating unit 52 is such that whereas detection of reflected waves by ultrasonic sensor 1 is carried out at intervals equal to a prescribed first wait time when it is estimated that the first state exists, detection of reflected waves by ultrasonic sensor 1 is carried out at intervals equal to a second wait time, which is shorter than the first wait time, when it is estimated that the second state exists.

In accordance with this constitution, estimating unit 52 switches detection frequency in two steps in correspondence to the amount of urine. More particularly, estimation of amount of urine is carried out at intervals equal to a first wait time when in a first state, for which the amount of urine is small; and estimation of amount of urine is carried out at intervals equal to a second wait time, which is shorter than the first wait time, when in a second state, for which the amount of urine is moderate.

Furthermore, estimating unit 52 determines when it is or will be time to urinate based on estimated amount of urine. That is, a urine amount estimation method further comprises an operation in which determination of when it is or will be time to urinate is carried out based on estimated amount of urine. For example, upon estimating that a third state, for which the amount of urine is large, exists, estimating unit 52 might determine that it is time to urinate.

Furthermore, urine amount estimation device 100 might be further provided with announcing unit 12 which announces that it is time to urinate, and estimating unit 52 might cause announcing unit 12 to operate when it has been determined that it is time to urinate.

In accordance with this constitution, when it has been determined by estimating unit 52 that it is time to urinate, announcing unit 12 announces this fact to the exterior. This permits the subject who is wearing ultrasonic sensor 1 and/or or another third party to know when it is time to urinate. For example, in a situation in which the subject is a person who, due to physical handicap, requires time before he or she is ready to use the toilet, it will be possible to alert the subject regarding urination before he or she feels a need to urinate. This will make it possible to prompt the subject to make preparations to use the toilet in early fashion. Furthermore, in a situation in which the subject is a person requiring nursing care, it will be possible to alert the person requiring nursing care regarding urination before he or she feels a need to urinate. This will make it possible for the nursing caregiver to have ample time in which to guide the person requiring nursing care to the toilet.

Moreover, after announcing that it is time to urinate, estimating unit 52 awaits feedback of the effect that urination has actually occurred, and performs correction of the time frame for announcing that it is time to urinate.

In accordance with this constitution, estimating unit 52 determines when it is or will be time to urinate based on the amount of urine. It so happens that because the storage capacity of the bladder varies from person to person, the amount of urine for which it should be determined that it is time to urinate also varies from person to person. After announcing that it is time to urinate, estimating unit 52 awaits feedback of the effect that urination has actually occurred and performs correction of the time frame for announcing that it is time to urinate. By so doing, the time frame for announcing will be corrected in correspondence to the subject. As a result, it will be possible to employ a more appropriate time frame for announcing when it is time to urinate.

More specifically, estimating unit 52 is constituted so as to await input of the fact that urination has actually occurred, and performs correction of the time frame for announcing based on the time from announcing that it is time to urinate until receipt of input of the fact that urination has actually occurred.

This makes it possible to perform correction of the time frame for announcing based on actual urination.

Furthermore, because ultrasonic sensor 1 is provided at the aforementioned protrusion 22, this improves closeness of contact with the skin (body surface) of the subject, and promotes entry of ultrasonic waves into the body. This makes it possible to increase detectability with respect to the small intestine and the bladder.

Moreover, as a result of causing protrusion 22 to be arranged at a comparatively low location on contacting surface 21, it is possible to reduce discomfort that might otherwise be imparted to the subject when urine amount estimation device 100 is worn. In other words, for detection of the small intestine and the bladder, it is preferred that ultrasonic waves be made to exit the lower abdomen from a comparatively low location thereof. However, if urine amount estimation device 100 is worn at too low a location on the lower abdomen, this will cause discomfort to be imparted to the subject. In this regard, causing protrusion 22 to be arranged at a comparatively low location on contacting surface 21 will make it possible for urine amount estimation device 100 to be arranged at a location that is as high as possible overall even while protrusion 22 is arranged at a low location on the lower abdomen. Because ultrasonic sensor 1 is incorporated within protrusion 22, it is possible to reduce discomfort to the subject while causing ultrasonic waves to exit the lower abdomen from a comparatively low location thereof.

### -Other Embodiments-

As indicated above, the foregoing embodiment has been described as an illustrative example of the art which is disclosed in the context of the present application. However, the art of the present disclosure is not limited hereto but may also be applied in the context of embodiments in which changes, substitutions, additions, omissions, and so forth have been carried out as appropriate. Furthermore, the respective constituent elements described at the foregoing embodiment may be combined to obtain new embodiments. Furthermore, the constituent elements appearing in the attached drawings and detailed description may include not only constituent elements which are essential for solution of problem(s) but also constituent elements which are not essential for solution of problem(s) but which have been included for purposes of illustrating the foregoing art by way of example. This being the case, the mere fact that such nonessential constituent elements appear in the attached drawings and detailed description should not serve as basis for presuming that such nonessential constituent elements are to be interpreted as essential constituent elements.

At the foregoing embodiment, a constitution such as the following may also be adopted.

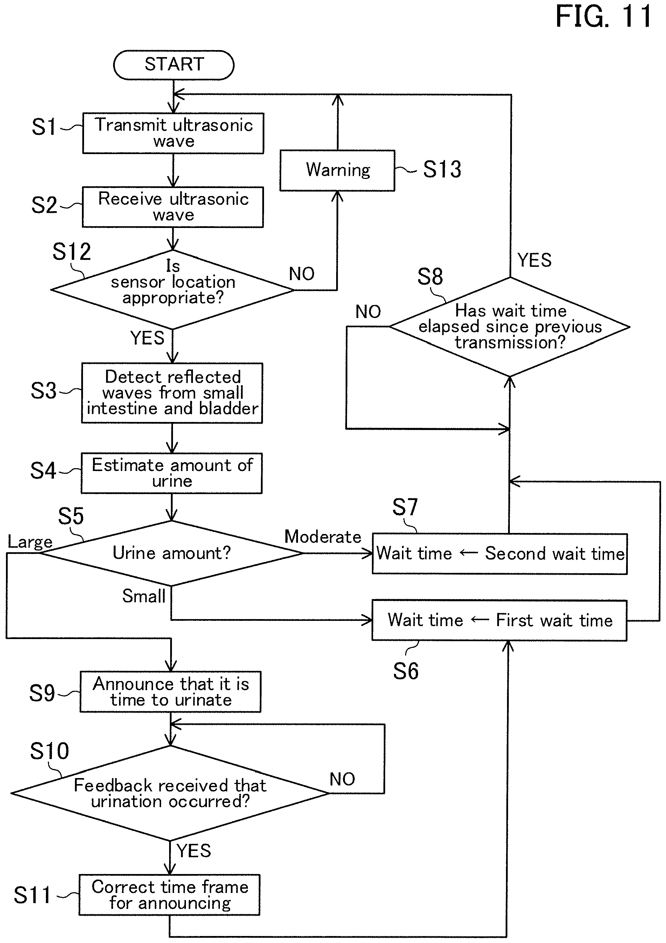

Urine amount estimation device 100 transmits ultrasonic waves (step S1), and, upon receipt of ultrasonic wave (step S2), carries out detection of reflected waves from the small intestine and the bladder (step S3). At such a time, urine amount estimation device 100 might, before carrying out detection of reflected waves from the small intestine and the bladder, carry out determination of whether the location at which ultrasonic sensor 1 is worn is suitable and/or determination of whether detection of the bladder 64 is possible. A flowchart applicable to such a situation is shown in FIG. 11.

At the flowchart shown in FIG. 11, following receipt of an ultrasonic wave at step S2, estimating unit 52 at step S12 determines whether the location at which ultrasonic sensor 1 is worn is suitable.

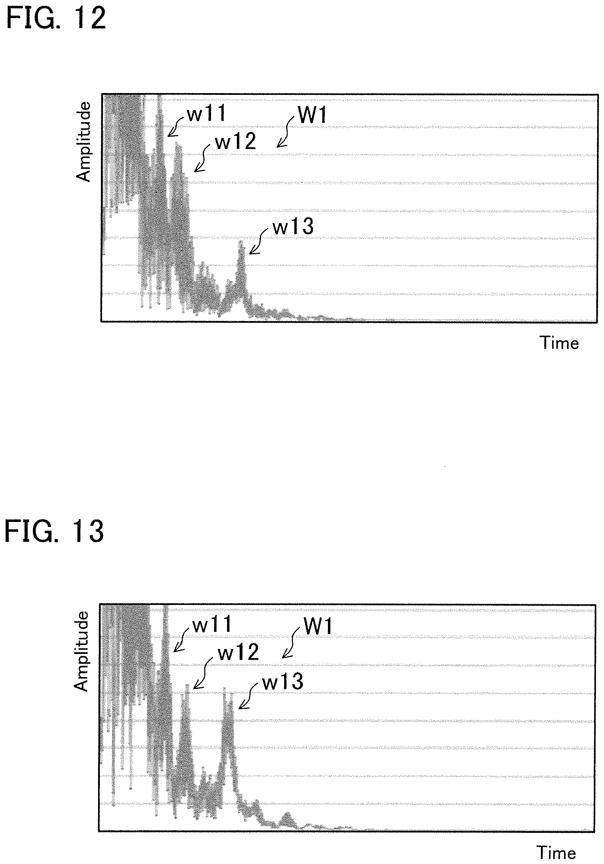

More particularly, estimating unit 52 determines whether the location at which ultrasonic sensor 1 is worn is suitable based on reflected wave W1 which comes immediately after the noise in the received signal. FIG. 12 shows a received signal that has been subjected to signal processing so as to permit detailed analysis of reflected wave W1. FIG. 13 shows a received signal that has been subjected to signal processing similar to that at FIG. 12, but at FIG. 13 the location at which ultrasonic sensor 1 is worn is higher than is the case at FIG. 12.

Because ultrasonic waves are reflected at boundaries between media having different acoustic impedances, an ultrasonic wave transmitted by ultrasonic sensor 1 is also reflected at the surfaces of subcutaneous fat 61, muscle 62, and fat 63. Upon performing detailed analysis of reflected wave W1, it is found that reflected wave W1 comprises reflected wave w11 from subcutaneous fat 61, reflected wave w12 from muscle 62, and reflected wave w13 from fat 63. In addition, upon comparing FIGS. 12 and 13, it is found that the times at which respective reflected waves w11 through w13 are received vary. As can be seen at FIG. 5, the thicknesses of subcutaneous fat 61, muscle 62, and fat 63 vary in correspondence to location in the vertical direction. It is possible to estimate the thicknesses of subcutaneous fat 61, muscle 62, and fat 63 based on the times at which reflected waves w11 through w13 are received, and so it is therefore also possible to estimate the location at which ultrasonic sensor 1 is worn.

If ultrasonic sensor 1 is worn at a location that is too high, there is a possibility that it may be impossible to detect the posterior wall 643 of the bladder 64 despite the fact that the amount of urine has increased. On the other hand, if ultrasonic sensor 1 is worn at a location that is too low, there is a possibility that the posterior wall 643 might be detected despite the fact that the amount of urine is small or that the ultrasonic wave transmitted from ultrasonic sensor 1 will be reflected by the pubic bone 610.

Estimating unit 52 therefore estimates the respective thicknesses of or the respective fractional percentages represented by subcutaneous fat 61, muscle 62, and fat 63 based on reflected waves w11 through w13, and based thereon determines whether the location at which ultrasonic sensor 1 is worn is suitable. Respective thicknesses of or respective fractional percentages represented by subcutaneous fat 61, muscle 62, and fat 63 when the location at which this is worn is suitable are determined in advance and stored at memory 11. Estimating unit 52 carries out the foregoing determination by comparing respective thicknesses of or respective fractional percentages represented by subcutaneous fat 61, muscle 62, and fat 63 estimated based on reflected waves w11 through w13 to respective thicknesses or respective fractional percentages stored at memory 11. Furthermore, estimating unit 52 may determine whether ultrasonic sensor 1 is worn at a location that is too low based on whether a reflected wave from the pubic bone 610 is present.

Whereas estimating unit 52 proceeds to the next step when the location at which ultrasonic sensor 1 is worn is suitable, estimating unit 52 warns the subject by way of announcing unit 12 (step S13), prompting correction of the location at which ultrasonic sensor 1 is worn, when the location at which ultrasonic sensor 1 is worn is unsuitable.

Note that because the respective thicknesses of or the respective fractional percentages represented by subcutaneous fat 61, muscle 62, and fat 63 vary greatly from person to person, even where it has been determined that the location at which ultrasonic sensor 1 is worn is unsuitable, it is also possible for estimating unit 52 to not do more than perhaps announce that fact to the subject before proceeding to the next step.

Furthermore, it is possible for whether the location at which ultrasonic sensor 1 is worn is suitable to be determined based on statuses of reflected waves when actual amount of urine is small and/or large. For example, when the amount of urine is small immediately following urination, whether the location at which ultrasonic sensor 1 is worn is suitable may be determined by ascertaining whether it is true that reflected wave W2 from the small intestine is observed and reflected wave W3 from the bladder is not observed. In addition thereto, when the need to urinate is felt, i.e., when the amount of urine is large, whether the location at which ultrasonic sensor 1 is worn is suitable may be determined by ascertaining whether it is true that reflected wave W2 from the small intestine is not observed and reflected wave W3 from the bladder is observed. Whether the location at which ultrasonic sensor 1 is worn is suitable may also be determined by ascertaining in this way whether appropriate reflected waves are observed when actual amounts of urine are small and/or large.

Furthermore, sensor(s) is/are not limited to ultrasonic sensor 1. Not only ultrasonic sensor(s) but any desired sensor(s) may be employed, so long as detection of the bladder and the small intestine is made possible thereby. And where ultrasonic sensor(s) is/are employed, constitution of the ultrasonic sensor(s) is not limited to the foregoing constitution. For example, an ultrasonic sensor having a plurality of transducers arranged in array-like fashion might be employed. Angles at which the ultrasonic waves exit the plurality of transducers may differ. For example, constitution might be such that a first transducer causes an ultrasonic wave to be irradiated from contacting surface 21 in perpendicular fashion, and a second transducer causes an ultrasonic wave to be irradiated so as to be directed in more downwardly inclined fashion than the first transducer. Where this is the case, reflected wave W3 from the bladder will become capable of being detected in order by the second transducer and then the first transducer as accumulation of urine proceeds, and reflected wave W3 from the bladder will stop being capable of being detected in order by the first transducer and then the second transducer as urination proceeds. This will make it possible for the amount of urine and the actual time to urinate to be estimated more precisely.

The manner in which urine amount estimation device 100 is worn is not limited to the foregoing. For example, contacting surface 21 might be formed so as to be a sticky-type adhesive surface, and contacting surface 21 might be made to adhere to the abdomen of the subject.

Whereas ultrasonic sensor 1 and device body 2 were constituted in integral fashion at urine amount estimation device 100, there is no limitation with respect thereto. For example, ultrasonic sensor 1 and device body 2 may be constituted as separate bodies, with only ultrasonic sensor 1 being worn by the subject, and device body 2 not necessarily being worn by the subject. Where this is the case, ultrasonic sensor 1 and device body 2 would communicate in wired or wireless fashion. Furthermore, the device(s) that are worn by the subject at least include ultrasonic sensor 1 but may also include other element(s). For example, transmitter 3 and receiver 4 may be separated from device body 2 and included among the device(s) that are worn by the subject. Moreover, signal processing unit 51 may be included among the device(s) that are worn by the subject. Moreover, it might be that controller 5 and memory 11 are included among the device(s) that are worn by the subject, and that only announcing unit 12 and input unit 13 are separated therefrom.

Note further that the device(s) that are not worn by the subject might be constituted by smartphone(s), PC(s), and/or other such communication terminal(s). For example, it might be that ultrasonic sensor 1, transmitter 3, receiver 4, controller 5, and memory 11 are formed in integral fashion, and announcing unit 12 and input unit 13 are constituted by communication terminal(s). In such a case, urine amount estimation device 100 might be provided with ultrasonic sensor 1, transmitter 3, receiver 4, controller 5, and memory 11; and communication terminal(s) in the possession of nursing caregiver(s) might, for example, function as announcing unit 12 and input unit 13. Urine amount estimation device 100 executes determination of when it is time to urinate as has been described above, and when it is time to urinate this fact is transmitted to communication terminal(s). The nursing caregiver receives the message that it is time to urinate, guides the person requiring nursing care to the toilet, and, when actual urination has occurred, transmits this fact to urine amount estimation device 100 by way of the communication terminal. Upon receiving report that urination has actually occurred, urine amount estimation device 100 performs correction of the time frame for announcing as has been described above.

Moreover, urine amount estimation device 100 might formed from ultrasonic sensor 1 and device body 2, and from smartphone(s), PC(s), and/or other such communication terminal(s) 200. Where this is the case, communication unit 14 and functional components other than those used for estimation of urine amount at estimating unit 52, signal processing unit 51, receiver 4, and transmitter 3 might be provided at device body 2; and those functional components used for estimation of urine amount at estimating unit 52, input unit 13, announcing unit 12, and memory 11 might be provided at communication terminal 200. In other words, from the transmission of ultrasonic waves and the reception of reflected waves produced thereby up until the subjecting of the received signal to signal processing might be carried out at ultrasonic sensor 1 and device body 2, the received signal that has been subjected to signal processing being transmitted from device body 2 to communication terminal 200. It might be the case that communication terminal 200 receives the received signal and stores it at memory 11, and also uses that received signal to estimate urine amount, predict when it is or will be time to urinate, and/or perform other such arithmetic processing, and carries out announcement and/or the like as necessary.

Moreover, urine amount estimation device 100 might be formed from ultrasonic sensor 1 and device body 2, and from server(s) and smartphone(s), PC(s), and/or other such communication terminal(s) 200. Where this is the case, communication unit 14 and functional components other than those used for estimation of urine amount at estimating unit 52, signal processing unit 51, receiver 4, and transmitter 3 might be provided at device body 2; those functional components used for estimation of urine amount at estimating unit 52 and memory 11 might be provided at server(s); and announcing unit 12 and input unit 13 might be provided at communication terminal(s) 200. In other words, from the transmission of ultrasonic waves and the reception of reflected waves produced thereby up until the subjecting of the received signal to signal processing might be carried out at ultrasonic sensor 1 and device body 2, the received signal that has been subjected to signal processing being transmitted from device body 2 to the server. It might be the case that the server receives the received signal and stores it at memory 11, and also uses that received signal to estimate urine amount, predict when it is or will be time to urinate, and/or perform other such arithmetic processing. In addition, the server transmits the urine amount and/or whether it is time to urinate to communication terminal 200; or, if announcement regarding urine amount and/or whether it is time to urinate is necessary, announces that fact to communication terminal 200. Communication terminal 200 receives information regarding urine amount and/or whether it is time to urinate from the server; and, where necessary, causes that content to be shown on a display and/or causes operation of an alarm or vibrator. With regard to feedback regarding urination, the fact that urination has occurred is transmitted from communication terminal 200 to the server as a result of actuation of communication terminal 200.

Urine amount estimation device 100 may estimate the amount of urine based on only either reflected wave W2 from the small intestine or reflected wave W3 from the bladder. For example, regardless of whether reflected wave W3 from the bladder is present, urine amount estimation device 100 might estimate that amount of urine is "small" when reflected wave W2 from the small intestine is observed, and might estimate that amount of urine is "large" when reflected wave W2 from the small intestine is not observed. Alternatively, regardless of whether reflected wave W2 from the small intestine is present, urine amount estimation device 100 might estimate that amount of urine is "small" when reflected wave W3 from the bladder is not observed, and might estimate that amount of urine is "large" when reflected wave W3 from the bladder is observed.

Furthermore, the amount of urine may be estimated not based on whether reflected wave W2 from the small intestine is present and/or whether reflected wave W3 from the bladder is present, but based on the magnitude of reflected wave W2 from the small intestine and/or the magnitude of reflected wave W3 from the bladder. As shown in FIGS. 6, 8, and 10, there is a tendency for reflected wave W2 from the small intestine to become smaller in accompaniment to increase in the amount of urine, and for reflected wave W3 from the bladder to become larger in accompaniment to increase in the amount of urine. For example, a determination threshold value might be set for the magnitude of reflected wave W2 from the small intestine, estimation of urine amount being carried out based on the relative magnitudes of said determination threshold value and the magnitude of reflected wave W2. Furthermore, a determination threshold value might be set for the magnitude of reflected wave W3 from the bladder, estimation of urine amount being carried out based on the relative magnitudes of said determination threshold value and the magnitude of reflected wave W3. Moreover, urine amount might be estimated by making an overall judgment with respect to the magnitude of reflected wave W2 from the small intestine and the magnitude of reflected wave W3 from the bladder. More specifically, estimating unit 52 might estimate that amount of urine is "small" when magnitude of reflected wave W2 from the small intestine is greater than or equal to a first determination threshold value and magnitude of reflected wave W3 from the bladder is less than a third determination threshold value; might estimate that amount of urine is "moderate" when magnitude of reflected wave W2 from the small intestine is less than the first determination threshold value and greater than or equal to a second determination threshold value (< the first determination threshold value) or magnitude of reflected wave W3 from the bladder is greater than or equal to a third determination threshold value and less than a fourth determination threshold value (> the third determination threshold value); and might estimate that amount of urine is "large" when magnitude of reflected wave W2 from the small intestine is less than the second determination threshold value and magnitude of reflected wave W3 from the bladder is greater than or equal to a fourth determination threshold value. In addition, estimating unit 52 may cause detection frequency to be greater when the amount of urine is "moderate" than when the amount of urine is "small," announcement of the fact that it is time to urinate being carried out when the amount of urine is "large."

Furthermore, urine amount estimation device 100 is not limited to devices that estimate amount of urine in three steps as being "small," "moderate," or "large." Urine amount estimation device 100 may estimate the amount of urine in two steps, in four or more steps, or in linear (continuous) fashion.

Moreover, estimating unit 52 may estimate the amount of urine through combination of whether reflected wave W2 from the small intestine is present, whether reflected wave W3 from the bladder is present, magnitude of reflected wave W2 from the small intestine, and/or magnitude of reflected wave W3 from the bladder.

For example, estimating unit 52 might estimate the amount of urine in three steps to broadly categorize this as "small," "moderate," or "large" through combination of whether reflected wave W2 from the small intestine is present and whether reflected wave W3 from the bladder is present as in the description above, and might perform detailed estimation of whether it is time to urinate based on magnitude of reflected wave W2 from the small intestine and/or magnitude of reflected wave W3 from the bladder. For example, it might be that estimating unit 52 is such that when reflected wave W2 is not observed and reflected wave W3 is observed, as a result of which the amount of urine is estimated to be "large," it is not determined on this basis alone that it is time to urinate, it being determined that it is time to urinate when magnitude of reflected wave W3 is above a prescribed determination threshold value, when magnitude of reflected wave W2 is below a prescribed determination threshold value, or when both of these conditions are satisfied. Where this is the case, reflected wave detection frequency may be varied in correspondence to the step represented by the amount of urine. More specifically, detection frequency is increased in correspondence to the stepwise increase in the amount of urine.

Furthermore, estimating unit 52 might adjust reflected wave detection frequency based on reflected wave W2, and might determine that it is time to urinate based on reflected wave W3. For example, estimating unit 52 might increase detection frequency so that it is greater when reflected wave W2 is small than when reflected wave W2 is large. At such time, estimating unit 52 might cause detection frequency to be increased in continuous fashion in correspondence to the magnitude of reflected wave W2, or might cause detection frequency to be increased in stepwise fashion in correspondence to the magnitude of reflected wave W2. On the other hand, estimating unit 52 might, regardless of the magnitude of reflected wave W2 and regardless of whether reflected wave W2 is present, determine that it is time to urinate when the magnitude of reflected wave W3 exceeds a prescribed determination threshold value.

Moreover, in a situation in which whether it is time to urinate is determined based on the magnitude of reflected wave W3, the reflected wave W3 determination threshold value for determining when it is time to urinate might be adjustable. Adjustment of this reflected wave W3 determination threshold value will make it possible to vary how many minutes immediately prior to urination it is that the determination that it is time to urinate is made. For example, if the user inputs that the determination that it is time to urinate should be made 5 minutes before urination, estimating unit 52 causes the amount of urine that is assumed to exist 5 minutes before urination to be set as the determination threshold value. Memory 11 stores the relationship between urine amount and time until urination using average bladder size as reference, and estimating unit 52 sets the determination threshold value so as to be the amount of urine which corresponds to the time that was input. Adjustment of this determination threshold value is equivalent to the aforementioned adjustment of the time frame for announcing when it is time to urinate. For this reason, in the event that the determination threshold value as set using average bladder size as reference differs from when it is time to urinate as input by the user, upon receiving input of feedback regarding urination as described above, estimating unit 52 performs correction of the determination threshold value. This makes it possible for estimating unit 52 to employ a more appropriate time frame for announcing when it is time to urinate.

Furthermore, correction of the determination threshold value might be carried out based not on input from input unit 13 but based on estimated value(s) for the amount of urine. For example, when the period at which determination of when it is time to urinate is carried out in repeated fashion is short, estimated values for amount of urine from the time that the amount of urine in the bladder is increasing and urination is carried out, up until the time that the amount of urine decreases are recorded in detail at memory 11. In such a situation, it will be possible to determine when urination actually occurred based on the estimated values for amount of urine that are recorded at memory 11, based on which it will be possible to calculate estimated value(s) of amount(s) of urine for which time(s) until urination is/are prescribed set time(s). For example, after announcing that it is time to urinate, the estimated value for the amount of urine reaches a maximum, the time thereafter when this suddenly decreases being the time at which urination actually occurs. In other words, the determination threshold value may be corrected based on the time from when it is announced that it is time to urinate until the estimated value for the amount of urine reaches a maximum, or until the amount of urine suddenly decreases. This will make it possible to more accurately correct the determination threshold value.

Furthermore, estimating unit 52 may when announcing that it is time to urinate also announce the approximate time until urination. With such a constitution, it is also possible to carry out correction of the predicted time until urination and the determination threshold value based on the estimated value for when urination actually occurred or will occur as described above.

Announcing unit 12 is not limited to vibrator(s). Announcing unit 12 may be alarm(s), lamp(s), or a combination thereof. Moreover, announcing unit 12 may take the form of display(s), it being possible for image(s) and/or animation(s) to be displayed in correspondence to the content to announced. For example, announcing unit 12 may cause image(s) to be shown on display(s) in correspondence to when it is time to urinate. More specifically, time until urination might be displayed in the form of an image depicting a person's face, the expression on the face being made to change so as to indicate increasingly intense anxiety as the time until urination grows shorter.

Furthermore, whereas transmitter 3 is such that the drive signal input to ultrasonic sensor 1 takes the form of a pulsed signal, the drive signal is not limited to pulsed signal(s). It is possible for the drive signal to be not a pulsed wave but a burst wave or the like.

Moreover, a Frequency Modulated Continuous Wave might be employed as the drive signal. In such case, estimating unit 52 performs frequency analysis on the received signal to detect reflected wave W2 from the small intestine and reflected wave W3 from the bladder. As technique(s) for performing frequency analysis, fast Fourier transform (FFT) may be employed, and/or the maximum entropy method (MEM) may be employed.

Furthermore, reflected wave W3 from the bladder which is detected by urine amount estimation device 100 is not limited to reflected wave(s) from the posterior wall 643 of the bladder 64. Depending on the location at which ultrasonic sensor 1 is worn, it is possible that reflected waves from wall portions other than the posterior wall 643, e.g., the superior wall 641 and so forth, may be able to be detected. Note, however, that based on considerations such as the angle at which ultrasonic waves enter the body from ultrasonic sensor 1 and the angle at which reflected waves exit from the wall(s) of the bladder 64, in a situation in which ultrasonic sensor 1 is worn on the abdomen it will be easier to detect reflected waves from the posterior wall 643 than from the superior wall 641 or the like.

Furthermore, whereas in the foregoing embodiment urine amount estimation device 100 predicts when it is or will be time to urinate based on estimated amount of urine, prediction of when it is or will be time to urinate need not be carried out. Urine amount estimation device 100 might only carry out estimation of urine amount alone, or might carry out estimation of urine amount and use that urine amount to predict event(s) other than when it is or will be time to urinate.

Moreover, urine amount estimation device 100 might be such that transmission and detection of ultrasonic waves are carried out when the subject assumes a prescribed posture. For example, it is possible that small intestine and bladder shape and location within the body may vary in correspondence to the posture of the subject. For this reason, causing the subject to assume a consistent posture when transmission and detection of ultrasonic waves are carried out will make it possible to more precisely estimate the amount of urine in the bladder. Urine amount estimation device 100 is therefore constituted such that when transmission and detection of ultrasonic waves are to be carried out the subject is prompted to assume a predetermined posture. For example, urine amount estimation device 100 might be constituted such that, before transmission and detection of ultrasonic waves are carried out, the fact that transmission and detection of ultrasonic waves are going to be carried out is announced to the subject. More specifically, estimating unit 52 might cause operation of announcing unit 12 and/or might carry out announcement(s) to registered communication terminal(s) 200 by way of communication unit 14. Note, however, that it is preferred that announcement of the fact that transmission and detection of ultrasonic waves are going to be carried out be performed in a manner other than that which is employed for announcing that it is time to urinate as described above. For example, in a situation in which announcing unit 12 is a vibrator, it is preferred that the vibrator be made to vibrate in a different manner depending on whether it is the fact that transmission and detection of ultrasonic waves are going to be carried out that is being announced or it is the fact that it is time to urinate that is being announced. Regarding the posture to be assumed when transmission and detection of ultrasonic waves are carried out, note that there is no need to be seated or to assume any other such particular posture, it being sufficient so long as posture is consistent each time that transmission and detection of ultrasonic waves are carried out in repeated fashion.

Also, urine amount estimation device 100 may be provided with a posture sensor that detects the posture of the subject. A gyrosensor may be cited as one example of a posture sensor. For example, estimating unit 52 may be such that transmission and detection of ultrasonic waves are carried out when the posture of the subject as detected by the gyrosensor is a prescribed measurement posture and it is time to transmit an ultrasonic wave as defined by the wait time as described above. Moreover, measurement posture(s) might be recumbent, seated, standing, and so forth, it being possible for the user to register any thereamong as desired.

Note, however, that there is little need for such detection of posture in the case of a subject who is a bedridden person requiring nursing care. For this reason, whether posture of the subject is to be one of the conditions for carrying out transmission and detection of ultrasonic waves might be made capable of being switched on and off.

Note further that whereas consistency of posture is preferred from the standpoint of estimation precision, it is not essential merely for estimation of the amount of urine.

### EXPLANATION OF REFERENCE NUMERALS

- 100: Urine amount estimation device
- 1: Ultrasonic sensor (sensor)
- 52: Estimating unit
- 200: Communication terminal

## Claims

1. A urine amount estimation device (100) comprising
a sensor (1) that is configured to transmit an ultrasonic wave into a body and to detect a reflected wave produced by said ultrasonic wave; and
an estimating unit (52) that is configured to estimate an amount of urine in a bladder (64) based on whether a reflected wave from the bladder (64) which is detected by the sensor (1) is present and/or based on a magnitude of the reflected wave from the bladder (64) which is detected by the sensor (1), wherein
the estimating unit (52) is configured to estimate the amount of the urine in the bladder (64) based on, in addition to the presence and/or the magnitude of the reflected wave from the bladder (64) which is detected by the sensor (1), whether a reflected wave from a small intestine (69) which is detected by the sensor (1) is present and/or a magnitude of the reflected wave from the small intestine (69) which is detected by the sensor (1).

2. The urine amount estimation device (100) according to claim 1 wherein
the estimating unit (52) is configured to
estimate that the amount of the urine in the bladder (64) is in a first state which is small when the reflected wave from the bladder (64) is not detected by the sensor (1) and the reflected wave from the small intestine (69) is detected by the sensor (1);
estimate that the amount of the urine in the bladder (64) is in a second state which is larger than the first state when the reflected wave from the bladder (64) is detected by the sensor (1) and the reflected wave from the small intestine (69) is detected by the sensor (1); and
estimate that the amount of the urine in the bladder (64) is in a third state which is larger than the second state when the reflected wave from the bladder (64) is detected by the sensor (1) and the reflected wave from the small intestine (69) is not detected by the sensor (1).

3. The urine amount estimation device (100) according to claim 1
wherein
the estimating unit (52) is configured to
adjust reflected wave detection frequency based on the reflected wave from the small intestine (69) which is detected by the sensor (1); and
estimate the amount of the urine in the bladder (64) based on the reflected wave from the bladder (64) which is detected by the sensor (1).

4. The urine amount estimation device (100) according to any one of claims 1 to 3 wherein
the estimating unit (52) is configured to
determine when it is or will be time to urinate based on an estimated amount of urine; and
determine when urination actually occurred based on a change of the estimated amount of urine to adjust, based on when urination actually occurred, determination reference for determining when it is or will be time to urinate.

5. The urine amount estimation device (100) according to any one of claims 1 to 4 wherein
the estimating unit (52) is configured to announce the fact that transmission and detection of ultrasonic waves are going to be carried out by the sensor (1) to the exterior.

6. The urine amount estimation device (100) according to any one of claims 1 to 4
further comprising a posture sensor that is configured to detect a posture of a subject who wears the sensor (1);
wherein the estimating unit (52) is configured to carry out transmission and detection of ultrasonic waves by the sensor (1) when the posture of the subject as detected by the posture sensor is a prescribed measurement posture.

7. A urine amount estimation method comprising
an operation in which an ultrasonic wave is transmitted by a sensor (1) into an interior of a body;
an operation in which a wave from the interior of the body produced by reflection of said ultrasonic wave is detected by the sensor (1); and
an operation in which an amount of urine in a bladder (64) is estimated based on, in addition to a presence and/or a magnitude of a reflected wave from the bladder (64) which is detected by the sensor (1), whether a reflected wave from a small intestine (69) which is detected by the sensor (1) is present and/or a magnitude of the reflected wave from the small intestine (69) which is detected by the sensor (1).

8. The urine amount estimation method according to claim 7 further comprising
an operation in which determination of when it is or will be time to urinate is carried out based on the estimated amount of the urine.

## Patentansprüche

1. Urinmengenbestimmungsvorrichtung (100), umfassend
einen Sensor (1), der zur Übertragung einer Ultraschallwelle in einen Körper und zur Erfassung einer durch die Ultraschallwelle erzeugten reflektieren Welle ausgebildet ist; und
eine Bestimmungseinheit (52), die zur Bestimmung einer Urinmenge in einer Blase (64) auf Basis des Auftretens einer von der Blase (64) reflektierten Welle, die durch den Sensor (1) erfassbar ist, und/oder auf Basis einer Stärke der von der Blase (64) reflektierten Welle, die durch den Sensor (1) erfassbar ist, ausgebildet ist, wobei die Bestimmungseinheit (52) zur Bestimmung der Menge des Urins in der Blase (64) auf Basis des Auftretens und/oder der Stärke der von der Blase (64) reflektierten Welle, die durch den Sensor (1) erfassbar ist, und zusätzlich auf Basis des Auftretens einer von einem Dünndarm (69) reflektierten Welle, die durch den Sensor (1) erfassbar ist, und/oder auf Basis einer Stärke der von dem Dünndarm (69) reflektierten Welle, die durch den Sensor (1) erfassbar ist, ausgebildet ist.

2. Urinmengenbestimmungsvorrichtung (100) nach Anspruch 1, wobei die Bestimmungseinheit (52) dazu ausgebildet ist,
zu bestimmen, dass sich die Menge des Urins in der Blase (64) in einem ersten Zustand befindet, der klein ist, wenn die von der Blase (64) reflektierte Welle nicht durch den Sensor (1) erfassbar ist und die von dem Dünndarm (69) reflektierte Welle durch den Sensor (1) erfassbar ist;
zu bestimmen, dass sich die Menge des Urins in der Blase (64) in einem zweiten Zustand befindet, der größer ist als der erste Zustand, wenn die von der Blase (64) reflektierte Welle durch den Sensor (1) erfassbar ist und die von dem Dünndarm (69) reflektierte Welle durch den Sensor (1) erfassbar ist; und
zu bestimmen, dass sich die Menge des Urins in der Blase (64) in einem dritten Zustand befindet, der größer ist als der zweite Zustand, wenn die von der Blase (64) reflektierte Welle durch den Sensor (1) erfassbar ist und die von dem Dünndarm (69) reflektierte Welle nicht durch den Sensor (1) erfassbar ist.

3. Urinmengenbestimmungsvorrichtung (100) nach Anspruch 1, wobei
die Bestimmungseinheit (52) dazu ausgebildet ist,
eine Frequenz der Erfassung der reflektierten Welle auf Basis der von dem Dünndarm (69) reflektierten Welle, die durch den Sensor (1) erfassbar ist, anzupassen; und
die Menge des Urins in der Blase (64) auf Basis der von der Blase (64) reflektierten Welle, die durch den Sensor (1) erfassbar ist, zu bestimmen.

4. Urinmengenbestimmungsvorrichtung (100) nach einem der Ansprüche 1 bis 3, wobei
die Bestimmungseinheit (52) dazu ausgebildet ist,
auf Basis einer bestimmten Urinmenge einen Zeitpunkt zu ermitteln, zu dem die Blase entleert werden sollte; und
auf Basis einer Veränderung der bestimmten Urinmenge zu ermitteln, wann die Blase tatsächlich entleert wurde, um auf Basis des Zeitpunkts, zu dem die Blase tatsächlich entleert wurde, eine Ermittlungsfrequenz der Ermittlung des Zeitpunkts zur Blasenentleerung anzupassen.

5. Urinmengenbestimmungsvorrichtung (100) nach einem der Ansprüche 1 bis 4, wobei
die Bestimmungseinheit (52) dazu ausgebildet ist, nach außen zu melden, dass durch den Sensor (1) eine Übertragung und Erfassung von Ultraschallwellen durchgeführt werden wird.

6. Urinmengenbestimmungsvorrichtung (100) nach einem der Ansprüche 1 bis 4, des Weiteren umfassend einen Körperhaltungssensor, der zur Erfassung einer Körperhaltung einer den Sensor (1) tragenden Person ausgebildet ist;
wobei die Bestimmungseinheit (52) dazu ausgebildet ist, eine Übertragung und Erfassung von Ultraschallwellen durch den Sensor (1) durchzuführen, wenn die von dem Körperhaltungssensor erfasste Körperhaltung der Person eine vorgeschriebene Messkörperhaltung ist.

7. Urinmengenbestimmungsverfahren, umfassend:
Übertragen einer Ultraschallwelle in ein Inneres eines Körpers durch einen Sensor (1);
Erfassen einer durch Reflexion der Ultraschallwelle erzeugten Welle aus dem Inneren des Körpers durch den Sensor (1); und
Bestimmen einer Urinmenge in einer Blase (64) auf Basis eines Auftretens und/oder einer Stärke einer von der Blase (64) reflektierten Welle, die durch den Sensor (1) erfassbar ist, und zusätzlich auf Basis des Auftretens einer von einem Dünndarm (69) reflektierten Welle, die durch den Sensor (1) erfassbar ist, und/oder auf Basis einer Stärke der von dem Dünndarm (69) reflektierten Welle, die durch den Sensor (1) erfassbar ist.

8. Urinmengenbestimmungsverfahren nach Anspruch 7, des Weiteren umfassend:
Ermitteln eines Zeitpunkts, zu dem die Blase entleert werden sollte, auf Basis der bestimmten Menge des Urins.

## Revendications

1. Dispositif (100) d'estimation de quantité d'urine comprenant
un capteur (1) qui est configuré pour transmettre une onde ultrasonique dans un corps et pour détecter une onde réfléchie produite par ladite onde ultrasonique ; et une unité d'estimation (52) qui est configurée pour estimer une quantité d'urine dans une vessie (64) sur la base de la présence d'une onde réfléchie par la vessie (64) et détectée par le capteur (1) et/ou sur la base d'une magnitude de l'onde réfléchie par la vessie (64) et détectée par le capteur (1), dans lequel
l'unité d'estimation (52) est configurée pour estimer la quantité de l'urine dans la vessie (64) sur la base de la présence et/ou la magnitude de l'onde réfléchie par la vessie (64) et détectée par le capteur (1) et, de plus, sur la base de la présence d'une onde réfléchie par un intestin grêle (69) et détectée par le capteur (1) et/ou d'une magnitude de l'onde réfléchie par l'intestin grêle (69) et détectée par le capteur (1).

2. Dispositif (100) d'estimation de quantité d'urine selon la revendication 1, dans lequel
l'unité d'estimation (52) est configurée pour
estimer que la quantité de l'urine dans la vessie (64) est dans un premier état qui est petit quand l'onde réfléchie par la vessie (64) n'est pas détectée par le capteur (1) et l'onde réfléchie par l'intestin grêle (69) est détectée par le capteur (1) ;
estimer que la quantité de l'urine dans la vessie (64) est dans un deuxième état qui est supérieur au premier état quand l'onde réfléchie par la vessie (64) est détectée par le capteur (1) et l'onde réfléchie par l'intestin grêle (69) est détectée par le capteur (1) ; et
estimer que la quantité de l'urine dans la vessie (64) est dans un troisième état qui est supérieur au deuxième état quand l'onde réfléchie par la vessie (64) est détectée par le capteur (1) et l'onde réfléchie par l'intestin grêle (69) n'est pas détectée par le capteur (1).

3. Dispositif (100) d'estimation de quantité d'urine selon la revendication 1, dans lequel
l'unité d'estimation (52) est configurée pour
ajuster une fréquence de détection de l'onde réfléchie sur la base de l'onde réfléchie par l'intestin grêle (69) et détectée par le capteur (1) ; et
estimer la quantité de l'urine dans la vessie (64) sur la base de l'onde réfléchie par la vessie (64) et détectée par le capteur (1).

4. Dispositif (100) d'estimation de quantité d'urine selon l'une quelconque des revendications 1 à 3, dans lequel
l'unité d'estimation (52) est configurée pour
déterminer quand il est ou sera temps d'uriner sur la base d'une quantité estimée d'urine ; et
déterminer quand l'urination s'est effectivement passée sur la base d'un changement de la quantité estimée d'urine afin d'ajuster, sur la base du moment où l'urination s'est effectivement passée, une référence de détermination pour déterminer quand il est ou sera temps d'uriner.

5. Dispositif (100) d'estimation de quantité d'urine selon l'une quelconque des revendications 1 à 4, dans lequel
l'unité d'estimation (52) est configurée pour annoncer vers l'extérieur le fait que le capteur (1) va transmettre et détecter des ondes ultrasoniques.

6. Dispositif (100) d'estimation de quantité d'urine selon l'une quelconque des revendications 1 à 4,
comprenant en outre un capteur de posture qui est configuré pour détecter une posture d'un sujet portant le capteur (1) ;
dans lequel l'unité d'estimation (52) est configurée pour transmettre et détecter des ondes ultrasoniques par le capteur (1) quand la posture du sujet détectée par le capteur de posture est une posture de mesure prescrite.

7. Procédé d'estimation de quantité d'urine comprenant
une opération dans laquelle une onde ultrasonique est transmise dans un intérieur d'un corps par un capteur (1) ;
une opération dans laquelle une onde venant de l'intérieur du corps et produit par la réflexion de ladite onde ultrasonique est détectée par le capteur (1) ; et
une opération dans laquelle une quantité d'urine dans une vessie (64) est estimée sur la base d'une présence et/ou d'une magnitude d'une onde réfléchie par la vessie (64) et détectée par le capteur (1) et, de plus, sur la base de la présence d'une onde réfléchie par un intestin grêle (69) et détectée par le capteur (1) et/ou d'une magnitude de l'onde réfléchie par l'intestin grêle (69) et détectée par le capteur (1).

8. Procédé d'estimation de quantité d'urine selon la revendication 7, comprenant en outre
une opération dans laquelle on détermine quand il est ou sera temps d'uriner sur la base de la quantité estimée de l'urine.
